# EUROPEAN PATENT APPLICATION

(11) **EP 4 115 834 A1**
(43) Date of publication of application: **11.01.2023**
(21) Application number: 22183542.4
(22) Date of filing: 07.07.2022
(51) Int. Cl.: A61B 18/14

(54) **BIASED ELECTRODES FOR IMPROVED TISSUE CONTACT AND CURRENT DELIVERY**

(30) Priority: 08.07.2021 US 202117371008
(71) Applicant: Biosense Webster (Israel) Ltd, Yokneam, 2066717 (IL)
(72) Inventor: GOVARI, Assaf, Yokneam 2066717 (IL); BEECKLER, Christopher Thomas, Irvine 92618 (US); KEYES, Joseph Thomas, Irvine 92618 (US); LICHTER, Justin George, Irvine 92618 (US)
(74) Representative: Carpmaels & Ransford LLP

(57) **Abstract**

A medical probe, including a flexible insertion tube having proximal and distal ends, and a basket assembly at the distal end of the flexible insertion tube. In embodiments of the present invention, the basket assembly includes a plurality of spines and a plurality of electrodes, each of the electrodes having a lumen therethrough fitting a given spine.

## Description

### CROSS-REFERENCE TO RELATED APPLICATION

This application claims the benefit of U.S. Provisional Patent Application 63/076,614, filed 10 September 2020, which is incorporated herein by reference.

### FIELD OF THE INVENTION

The present invention relates generally to medical probes, and specifically to fabricating biased electrodes for a basket catheter.

### BACKGROUND OF THE INVENTION

Arrhythmias are abnormal heart rhythms that are typically caused by a small area of cardiac tissue that produces irregular heartbeats. Cardiac ablation is a medical procedure that can be performed to treat an arrhythmia by destroying the area of the cardiac tissue causing the irregular heartbeats. Some medical systems use irreversible electroporation (IRE) to ablate cardiac tissue. IRE is a nonthermal ablation method based on the unrecoverable permeabilization of cell membranes caused by short pulses of high voltage delivered to the tissue.

U.S. Patent Application 2013/0282084 to Mathur et al., describes an apparatus for treatment of in-stent restenosis. The apparatus includes an elongate flexible catheter body with a radially expandable structure comprising a plurality of electrodes or other electrosurgical energy delivery surfaces to engage tissue when the structure expands. The electrodes may be fabricated as part of struts that form a basket. In some embodiments, the electrodes can be wider than the struts.

U.S. Patent 6,569,162 to He describes a design for a passively self-cooled ablation electrode. The electrode includes a greater surface area that allows electrode to dissipate heat to the blood pool more effectively and increased thermal mass, thereby improving heat transfer between the electrode and tissue for more controlled heating and ablation of the tissue. The electrode comprises a substantially solid electrode body with thick walls that results in increased thermal mass and thermal conductivity. In one embodiment, the electrode may have a substantially curved exterior surface at the distal end of the electrode body, for use a tip electrode, or a central lumen extending through the electrode body, for use a ring electrode. In another embodiment, the plurality of projections integrally formed within the exterior surface of the electrode extend substantially parallel to the axis of the electrode to form a plurality of axial fins.

U.S. Patent 6,582,429 to Krishnan et al., describes a design for an ablation catheter with covered electrodes. The catheter includes a shaft having a curved distal-end region with an inner surface and an outer surface and a plurality of band electrodes positioned at the distal end of the shaft. The configuration of the electrodes may comprise twelve band electrodes arranged in a substantially linear array along the distal segment of the catheter sheath. In some embodiments, the electrodes may comprise a tip electrode.

U.S. Patent 8,475,450 to Govari et al., describes a dual-purpose lasso catheter with irrigation. The lasso catheter includes electrodes that that bulge above the outer surface of the electrodes in order to increase the surface area that is in contact with heart tissue, and thus reduce the electrical resistance when the electrodes are used for ablation.

The description above is presented as a general overview of related art in this field and should not be construed as an admission that any of the information it contains constitutes prior art against the present patent application.

### SUMMARY OF THE INVENTION

There is provided, in accordance with an embodiment of the present invention, a surgical imaging system, including a flexible insertion tube having proximal and distal ends, a basket assembly at the distal end of the flexible insertion tube, the basket assembly including a plurality of spines, and a plurality of electrodes, each of the electrodes having a lumen therethrough fitting a given spine.

In one embodiment, the spines have respective outer sides and inner sides, and each given electrode includes a conductive material biased towards the outer side of is respective spine.

In another embodiment, the spines have respective outer sides and inner sides, and each given electrode also has a notch between the lumen and the inner side of its respective spine.

In some embodiments, each given spine includes a wire affixed to the inner side of the given spine, and the notch includes solder material forming an electrical connection between the each given electrode and the wire.

In an additional embodiment, the surgical imaging system also includes an adhesive material applied to an inner surface of each given electrode, and the inner surface is defined by the lumen of the each given electrode.

In a further embodiment, each of the electrodes has one or more smoothed edges.

In a supplemental embodiment, the medical probe also includes a set of spray ports configured to deliver an irrigation fluid to the electrodes.

In another embodiment, the medical probe also includes a set of spray ports configured to deliver an irrigation fluid between the spines.

There is also provided, in accordance with an embodiment of the present invention, a method including providing a medical probe having a plurality of spines, providing an extruded tube of a conductive material, performing a cutting operation on the extruded tube so as to form an electrode body, and fitting the formed electrode body around a given spine.

In one embodiment, the plurality of spines forms a basket assembly at a distal end of the medical probe.

In another embodiment, the cutting operation forms sharp edges on the electrode body, and the method also includes smoothing out the sharp edges.

In an additional embodiment, the extruded tube defines a lumen, wherein the tube is not radially symmetric about the lumen.

In a further embodiment, the given spine has an outer side and an inner side, wherein fitting the formed electrode body on the given spine includes biasing the electrode body towards the outer side of the given spine.

In a supplemental embodiment, the given spine has an outer side and an inner side, wherein the cutting operation includes cutting a notch between the lumen and the inner side of the given spine.

In some embodiments, the given spine includes a wire affixed to the inner side of the given spine, and the method also includes filling the notch with solder so as to form an electrical connection between the wire and the electrode body.

In an additional embodiment, fitting the formed electrode body around the given spine includes forming an adhesive bond between the electrode body and the given spine.

In a further embodiment, the method also includes providing a set of spray ports configured to deliver an irrigation fluid to the electrodes.

In another embodiment, the method also includes providing a set of spray ports configured to deliver an irrigation fluid between the spines.

### BRIEF DESCRIPTION OF THE DRAWINGS

The disclosure is herein described, by way of example only, with reference to the accompanying drawings, wherein:
Figure 1 is a schematic pictorial illustration of a medical system 20 comprising a medical probe whose distal end comprises a basket assembly with biased electrodes, in accordance with an embodiment of the present invention;
Figure 2 is a schematic pictorial illustration showing angled views of a given biased electrode, in accordance with an embodiment of the present invention;
Figures 3A and 3B, also referred to herein collectively as Figure 3 are schematic pictorial illustrations of the distal end of the medical probe, in accordance with an embodiment of the present invention;
Figure 4 is a flow diagram that schematically illustrates a method of fabricating the biased electrodes and fitting the biased electrodes to spines of the basket assembly, in accordance with an embodiment of the present invention;
Figures 5A-5C, also referred to herein collectively as Figure 5 are schematic pictorial illustrations showing the fabrication of a given biased electrode from an extruded tube of a conductive material, in accordance with an embodiment of the present invention; and
Figures 6 and 7 are longitudinal views of fitting the lumen of a given electrode body on a given spine so that the given electrode body is biased toward an outer side of the given spine, in accordance with an embodiment of the present invention.

### DETAILED DESCRIPTION OF EMBODIMENTS

### OVERVIEW

To deliver pulsed field ablation (PFA) in an IRE (irreversible electroporation) procedure, electrodes should contact the tissue being ablated with as large a surface area as possible. Embodiments of the present invention provide a medical probe with a basket assembly comprising electrodes that are biased in cross section so that there is more material on the tissue contacting side, while minimizing unnecessary material on the non-tissue contacting side. As described hereinbelow, the medical probe includes a flexible insertion tube having proximal and distal ends, and a basket assembly at the distal end of the flexible insertion tube. The basket assembly comprises a plurality of spines and a plurality of electrodes, each given electrode having a lumen therethrough fitting a given spine.

Biasing the electrodes so that there is more material on the tissue contacting side allows the basket assembly of a medical probe implementing embodiments of the present invention to collapse into a small sheath. Another aspect of this electrode design ensures that the bias (i.e., the protrusion) is atraumatic. In some embodiments, the atraumaticity is implemented by smoothing out any sharp edges in the electrodes. Smoothing out sharp edges prevents any damage to tissue or the sheath, and helps prevent high current density that may result in arcing.

### SYSTEM DESCRIPTION

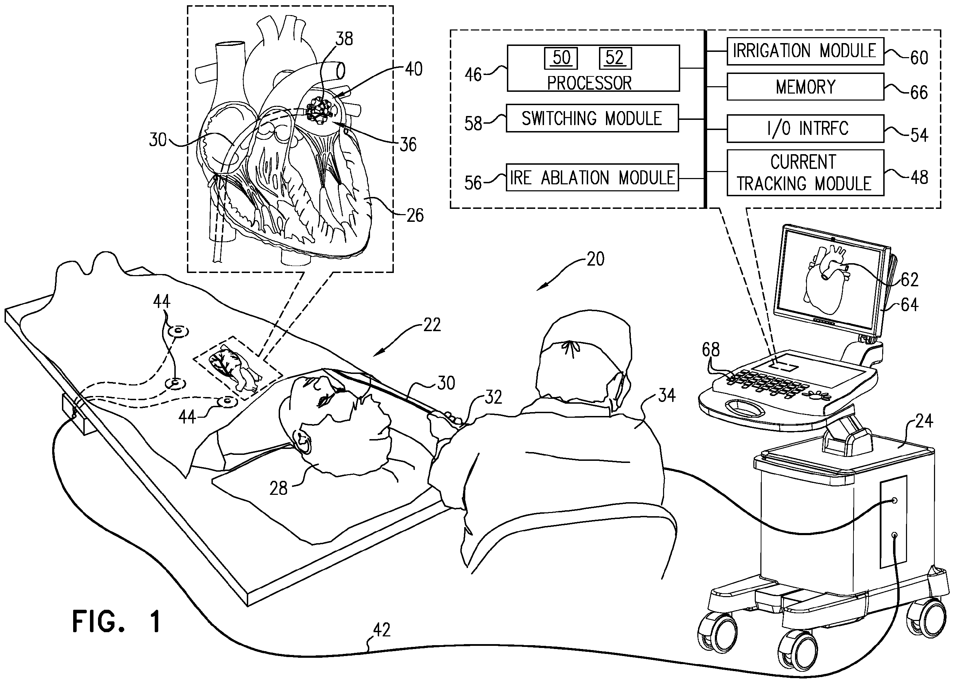

Figure 1 is a schematic, pictorial illustration of a medical system 20 comprising a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, *mutatis mutandis*, for other therapeutic and/or diagnostic purposes in the heart or in other body organs.

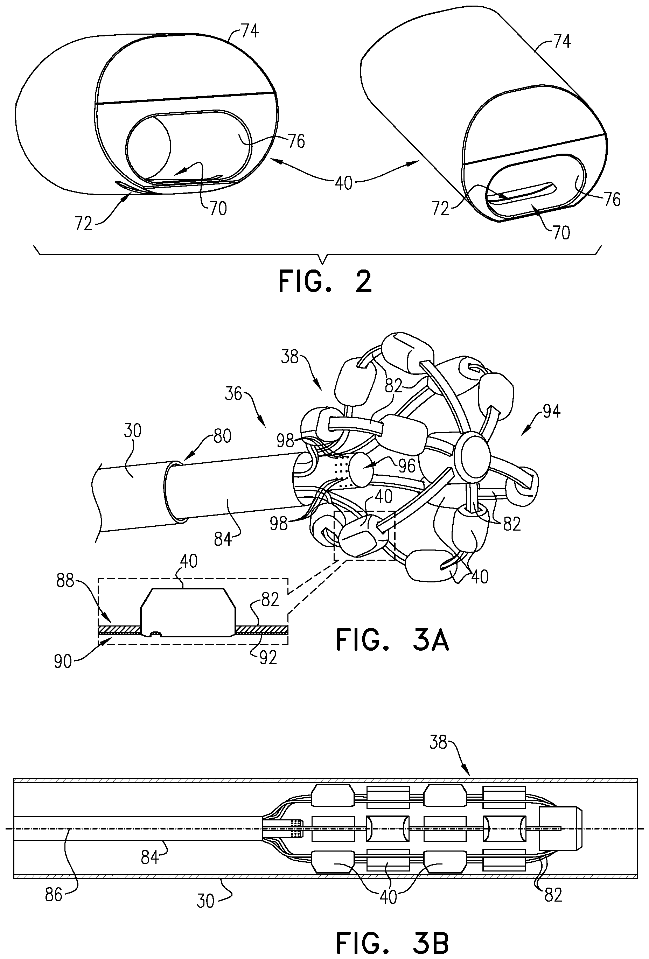

Probe 22 comprises a flexible insertion tube 30 and a handle 32 coupled to a proximal end of the insertion tube. During a medical procedure, a medical professional 34 can insert probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 affixed to distal end 36. Basket assembly 38 comprises a set of electrodes 40 (also referred to herein as electrode bodies 40) affixed to a plurality of spines, as described in the description referencing Figures 3, 6 and 7 hereinbelow.

To start performing a medical procedure such as irreversible electroporation (IRE) ablation, medical professional 34 can manipulate handle 32 to position distal end 36 so that electrodes 40 engage cardiac tissue at a desired location or locations.

In the configuration shown in Figure 1, control console 24 is connected, by a cable 42, to body surface electrodes, which typically comprise adhesive skin patches 44 that are affixed to patient 28. Control console 24 comprises a processor 46 that, in conjunction with a current tracking module 48, determines location coordinates of distal end 36 inside heart 26 based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with current tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are (also) configured to deliver IRE ablation energy to tissue in heart 26, configuring electrodes 40 to deliver any other type of ablation energy to tissue in any body cavity is considered to be within the spirit and scope of the present invention.

Processor 46 may comprise real-time noise reduction circuitry 50 typically configured as a field programmable gate array (FPGA), followed by an analog-to-digital (A/D) signal conversion integrated circuit 52. The processor can be programmed to perform one or more algorithms disclosed herein, each of the one or more algorithms comprising steps described hereinbelow. The processor uses circuitry 50 and circuit 52 as well as features of modules which are described in more detail below, in order to perform the one or more algorithms.

Although the medical system shown in Figure 1 uses impedance or current-based sensing to measure a location of distal end 36, other location tracking techniques may be used (e.g., techniques using magnetic-based sensors). Impedance and current-based location tracking techniques are described, for example, in U.S. Patents 5,983,126, 6,456,864 and 5,944,022. The methods of location sensing described hereinabove are implemented in the above-mentioned CARTO^{®} system and are described in detail in the patents cited above.

Control console 24 also comprises an input/output (I/O) communications interface 54 that enables control console 24 to transfer signals from, and/or transfer signals to electrodes 40 and adhesive skin patches 44. In the configuration shown in Figure 1, control console 24 additionally comprises an IRE ablation module 56 and a switching module 58.

IRE ablation module 56 is configured to generate IRE pulses comprising peak power in the range of tens of kilowatts. As described hereinbelow, medical system 20 performs IRE ablation by delivering IRE pulses to pairs of electrodes 40. In some embodiments, a given pair of the electrodes comprises two sets of electrodes 40 with each of the sets having at least one electrode 40. Using switching module 58, IRE ablation module 56 can deliver one or more IRE pulses independently to each of the pairs of the electrodes.

In order to dissipate the heat and to improve the efficiency of the ablation process, system 20 supplies irrigation fluid (e.g., a saline solution) to distal end 36 via a channel (not shown) in insertion tube 30. Control console 24 comprises an irrigation module 60 to monitor and control irrigation parameters, such as the pressure and the temperature of the irrigation fluid.

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may comprise any suitable volatile and/or non-volatile memory, such as random-access memory or a hard disk drive.

In some embodiments, medical professional 34 can manipulate map 62 using one or more input devices 68. In alternative embodiments, display 64 may comprise a touchscreen that can be configured to accept inputs from medical professional 34, in addition to presenting map 62.

Figure 2 is a schematic pictorial illustration showing angled views of a given electrode 40, in accordance with an embodiment of the present invention. Each electrode 40 is fabricated from a biocompatible electrically conductive material, has an electrode lumen 70 therethrough, and has a notch 72 that extends from an outer surface 74 of the electrode to the lumen. In addition to outer surface 74, each given electrode 40 has an inner surface 76 defined by its respective lumen 70.

Figures 3A and 3B, also referred to herein collectively as Figure 3, are schematic pictorial illustrations of distal end 36 comprising basket assembly 38, in accordance with an embodiment of the present invention. Figure 3A shows basket assembly 38 in an expanded configuration when unconstrained, such as by being advanced out of an insertion tube lumen 80 at distal end 36, and Figure 3B shows the basket assembly in a collapsed configuration within insertion tube 30. As shown in Figure 3B, the outward bias of electrodes 40 enable spines 82 to lay flush with shaft 84 when basket assembly 38 is collapsed within insertion tube 30.

By way of example, as shown in Figure 3A, basket assembly 38 comprises a plurality of flexible spines 82 that are formed at the end of a tubular shaft 84 and are connected at their proximal and distal ends. During a medical procedure, medical professional 34 can deploy basket assembly 38 by extending tubular shaft 84 from insertion tube 30. Spines 82 may have elliptical (e.g., circular) or rectangular (that may appear to be flat) cross-sections, and typically comprise a flexible, resilient material (e.g., a shape-memory alloy such nickel-titanium, also known as Nitinol). In its expanded configuration, basket assembly 38 has an expanded arrangement (Figure 3A) wherein spines 82 bow radially outwardly and a collapsed arrangement (Figure 3B) wherein the spines are arranged generally along a longitudinal axis 86 of insertion tube 30.

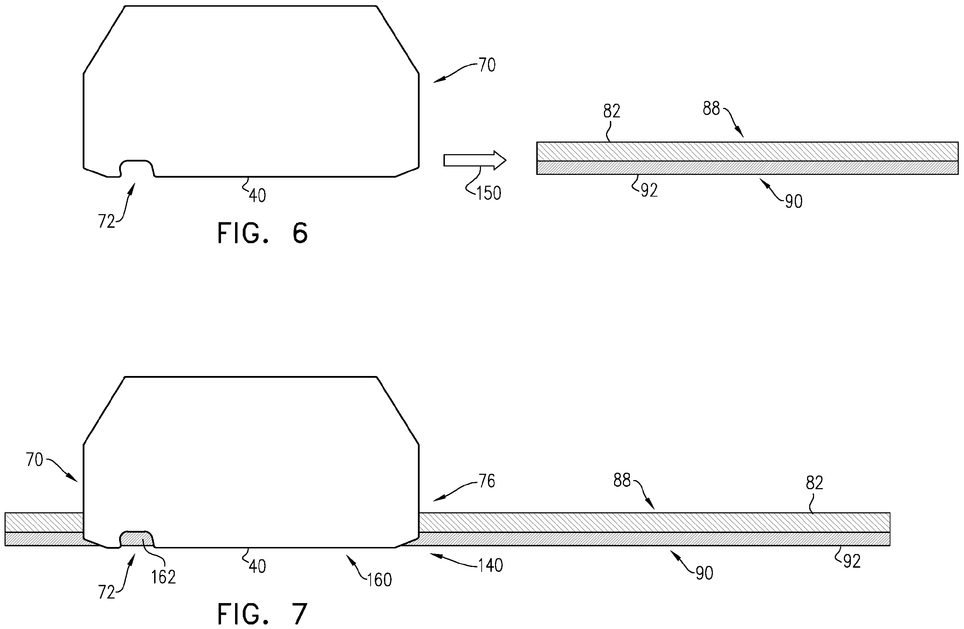

In the configuration shown in Figure 3, one or more electrodes 40 are threaded on to each given spine 82 so as to fit the electrodes to the spines. Fitting a given electrode 40 to a given spine 82 is described in the description referencing Figures 6 and 7 hereinbelow.

In embodiments described herein, electrodes 40 can be configured to deliver ablation energy to tissue in heart 26. In addition to using electrodes 40 to deliver ablation energy, the electrodes can also be used to determine the location of basket assembly 38 and/or to measure a physiological property such as local surface electrical potentials at respective locations on tissue in heart 26.

Each spine 82 has an outer side 88 and an inner side 90. In embodiments of the present invention, for a given electrode 40 fitted to a given spine 82, the given spine is planar at the given electrode, wherein the plane divides the given electrode asymmetrically so that there is more conductive material on the outer side (of the plane) than on the inner side. In these embodiments, electrodes 40 (i.e., when fitted to spines 82) are biased towards the outer sides of the spines 82, as there is a greater surface area of the given electrode on the outer side compared to the surface area of the given electrode on the inner side.

Examples of materials ideally suited for forming electrodes 40 include gold, platinum and palladium (and their respective alloys). These materials also have very high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 26.

Probe 22 also comprises a set of wires 92 that couple IRE ablation module 56 to electrodes 40. In some embodiments each spine 82 comprises a given wire 92 affixed to its inner side 90. Connecting wires 92 to electrodes 40 is described in the description referencing Figure 7 hereinbelow.

Basket assembly 38 has a distal end 94 and comprises a stem 96 that extends longitudinally from a distal end of shaft 84 towards distal end 94. As described supra, control console 24 comprises irrigation module 60 that delivers irrigation fluid to distal end 36. Stem 96 comprises multiple spray ports 98, wherein each given spray port 98 is angled to aim delivery of the irrigation fluid to either a given electrode 40 or to tissue in heart 26 (i.e., by aiming the delivery between two adjacent spines 82).

By biasing electrodes 40 to outer side 88, the electrodes deliver more ablation energy from the portion of the electrodes outer side of the spines (i.e., significantly more than the ablation energy delivered from the portion of the electrodes on the inner side of the spines). Since electrodes 40 do not comprise spray ports that deliver irrigation fluid, the configuration described hereinabove enables heat to be transferred from the tissue (i.e., during an ablation procedure) to the portion of the electrodes on the inner side of the spines, and the electrodes can be cooled by aiming the irrigation fluid, via spray ports 98, at the portion of the electrodes on the inner side of the spines.

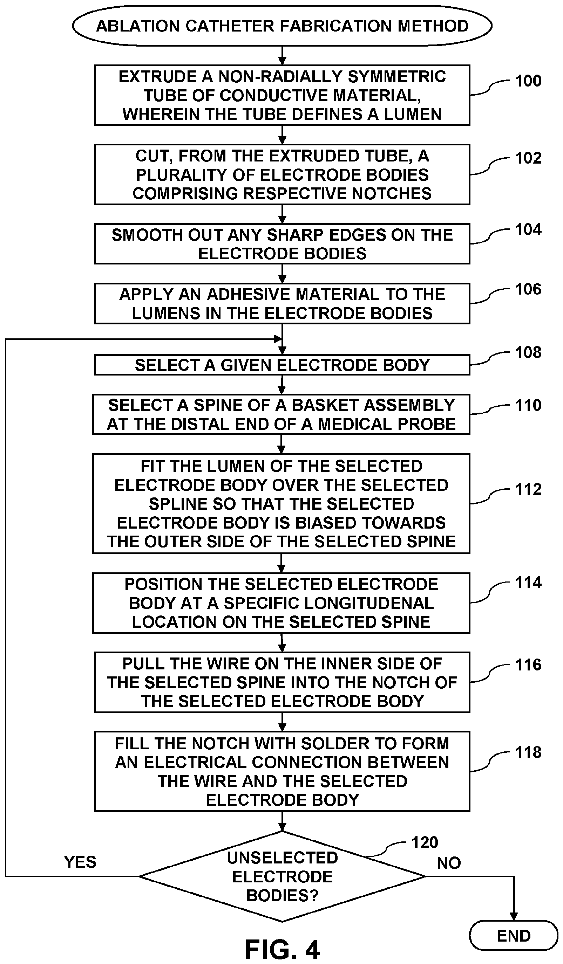

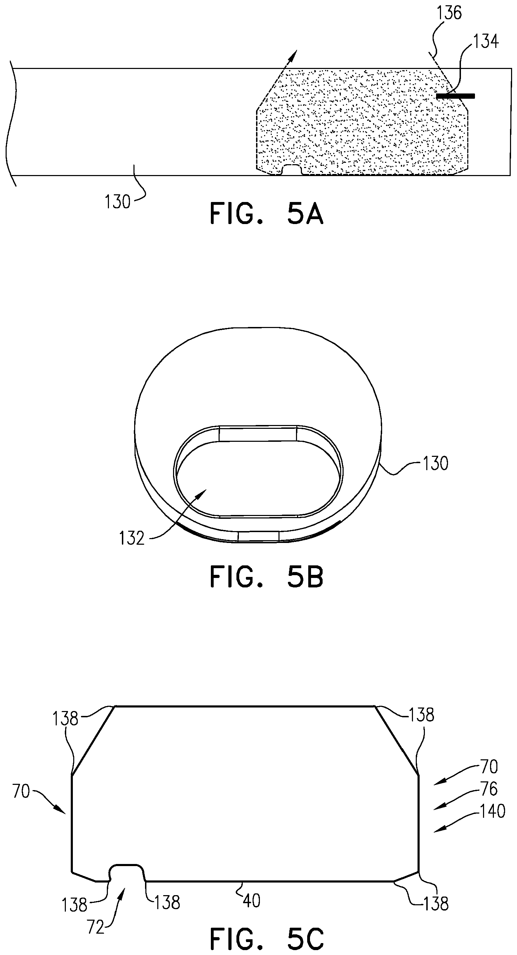

Figure 4 is a flow diagram that schematically illustrates a method of fabricating electrodes 40 and fitting the electrodes to spines 82, and Figures 5A-5C, also referred to herein collectively as Figure 5, are schematic pictorial illustrations showing the fabrication of a given electrode 40 from an extruded tube 130 of a conductive material, in accordance with an embodiment of the present invention. Figure 5A shows a longitudinal view of extruded tube 130, Figure 5B shows a cross-sectional latitudinal view of the tube 130 that defines a tube lumen 132, and Figure 5C shows a longitudinal view of a given extruded electrode body 40.

In an extrusion step 100, a manufacturer (not shown) extrudes non-radially symmetric tube 130 of conductive material, as shown in the cross-sectional latitudinal view of the tube in Figure 5B. For example, tube 130 may have a height of 0.04 inches (± 0.003 inches) and a width of 0.048 inches (± 0.003 inches), the lumen may have a height of 0.017 inches (± 0.001 inches) and a width of 0.032 inches (±0.002 inches), the thickness of the conductive material below the lumen may be 0.003 inches (± 0.001 inches), and the thickness of the conductive material above the lumen may be approximately 0.02 inches.

In a fabrication step 102, the manufacturer uses a cutting instrument to cut a plurality of electrode bodies 40 (Figure 5C) from tube 130. In some embodiments, each electrode may have a length of approximately 0.08 inches.

In the example shown in Figure 5A, the manufacturer can use a cutting instrument such as an electrical discharging machining (EDM) wire 134 to fabricate the electrode bodies from tube 130. In some embodiments, the manufacturer can use EDM wire 134 to fabricate each of the electrode bodies (including notch 72) in a single cutting operation, as shown by a cutting path 136.

Fabrication step 102 may create sharp edges 138 (Figure 5C) on electrode bodies 40. If medical system 20 is used for performing high-energy ablation procedures such as IRE ablation, applying the high ablation energy to electrodes 40 may result in arcing from edges 138.

In a smoothing step 104, the manufacturer smooths out edges 138 on the fabricated electrode bodies. Examples of smoothing processes that the manufacturer can use include, but are not limited to, tumbling/rumbling and barreling.

In an application step 106, the manufacturer applies an adhesive 140 to inner surfaces 76 of electrode bodies 40.

In a first selection step 108, the manufacturer selects a given fabricated electrode body 40, and in a second selection step 110, the manufacturer selects a given spine 82 for the selected electrode body.

In a fitting step 112, the manufacturer fits the lumen of selected electrode body to the selected spine so that the selected electrode body is biased toward the outer side of the selected spine.

Figures 6 and 7 are longitudinal views of fitting lumen 70 of a given electrode body 40 on a given spine 82 (as indicated by an arrow 150 in Figure 6), so that the given electrode body is biased toward outer side 88 of the given spine, in accordance with an embodiment of the present invention.

In a positioning step 114, the manufacturer positions the selected electrode body at a specific longitudinal location 160 on the selected spine. In some embodiments, positioning the selected electrode body at the desired location enables adhesive 140 to set, thereby forming an adhesive bond between the selected electrode body and the selected spine. One advantage of the configuration of electrodes 40 is that if the adhesive bond breaks between a given electrode 40 and a given spine 82, the lumen of the given spine will still contain the given electrode thereby preventing the given electrode from "breaking off" basket assembly 38.

In a pull step 116, the manufacturer pulls (e.g., "fishhooks") the wire from the inner side of the selected electrode body into the notch of the selected electrode body, and in a connection step 118, the manufacturer fills the notch (i.e., containing the pulled wire) with a solder material 162 (e.g., a gold-tin alloy), thereby forming an electrical connection between the wire and the selected electrode body (and thereby forming an electrical connection between the selected electrode body and ablation module 56).

Finally, in a decision step 120 if there are still any electrode bodies 40 that have not yet been selected for basket assembly 38 (i.e., in step 108), then the method continues with step 108, where the manufacturer can select a previously unselected electrode body 40. The method ends when there are no more unselected electrode bodies 40 for basket assembly 38. For example, the basket assembly shown in Figure 2 comprises twelve electrodes 40 (i.e., six spines 82 with two electrode bodies fit to each of the spines). Therefore, the method ends upon completing to fix two electrodes to each spine 82.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A medical probe, comprising:
a flexible insertion tube having proximal and distal ends;
a basket assembly at the distal end of the flexible insertion tube, the basket assembly comprising a plurality of spines; and
a plurality of electrodes, each of the electrodes having a lumen therethrough fitting a given spine.

2. The medical probe according to claim 1, wherein the spines have respective outer sides and inner sides, and wherein each given electrode comprises a conductive material biased towards the outer side of its respective spine.

3. The medical probe according to claim 1, wherein the spines have respective outer sides and inner sides, and wherein each given electrode has a notch between the lumen and the inner side of its respective spine.

4. The medical probe according to claim 3, wherein each given spine comprises a wire affixed to the inner side of the given spine, and wherein the notch comprises solder material forming an electrical connection between the each given electrode and the wire.

5. The medical probe according to claim 1, and further comprising an adhesive material applied to an inner surface of each given electrode, and wherein the inner surface is defined by the lumen of the each given electrode.

6. The medical probe according to claim 1, wherein each of the electrodes has one or more smoothed edges.

7. The medical probe according to claim 1, and further comprising a set of spray ports configured to deliver an irrigation fluid to the electrodes, or between the spines.

8. A method, comprising:
providing a medical probe having a plurality of spines;
providing an extruded tube of a conductive material;
performing a cutting operation on the extruded tube so as to form an electrode body; and
fitting the formed electrode body around a given spine.

9. The method according to claim 8, wherein the plurality of spines forms a basket assembly at a distal end of the medical probe.

10. The method according to claim 8, wherein the cutting operation forms sharp edges on the electrode body, and further comprising smoothing out the sharp edges.

11. The method according to claim 8, wherein the extruded tube defines a lumen, and wherein the tube is not radially symmetric about the lumen.

12. The method according to claim 8, wherein the given spine has an outer side and an inner side, and wherein fitting the formed electrode body on the given spine comprises biasing the electrode body towards the outer side of the given spine.

13. The method according to claim 8, wherein the given spine has an outer side and an inner side, and wherein the cutting operation comprises cutting a notch between the lumen and the inner side of the given spine, optionally wherein the given spine comprises a wire affixed to the inner side of the given spine, and further comprising filling the notch with solder so as to form an electrical connection between the wire and the electrode body.

14. The method according to claim 8, wherein fitting the formed electrode body around the given spine comprises forming an adhesive bond between the electrode body and the given spine.

15. The method according to claim 8, and further comprising providing a set of spray ports configured to deliver an irrigation fluid to the electrodes, or between the spines.
